# EUROPÄISCHE PATENTSCHRIFT

(11) **EP 2 804 649 B1**
(45) Veröffentlichungstag und Bekanntmachung des Hinweises auf die Patenterteilung: **22.11.2017**
(21) Anmeldenummer: 14706770.6
(22) Anmeldetag: 15.01.2014
(51) Int. Cl.: A61M 13/00

(54) **VORRICHTUNG ZUR GASERWÄRMUNG UND -BEFEUCHTUNG MITTELS MAGNETISCHER INDUKTION FÜR DIE LAPAROSKOPIE**
DEVICE FOR GAS HEATING AND HUMIDIFICATION BY MEANS OF MAGNETIC INDUCTION FOR LAPAROSCOPY
DISPOSITIF DE CHAUFFAGE ET D'HUMIDIFICATION DE GAZ PAR INDUCTION MAGNÉTIQUE POUR LA LAPAROSCOPIE

(30) Priorität: 15.01.2013 DE 102013000491
(43) Veröffentlichungstag der Anmeldung: 26.11.2014
(73) Patentinhaber: W.O.M. World of Medicine GmbH, 10587 Berlin (DE)
(72) Erfinder: MÜLLER, Bernd, 10247 Berlin (DE); KÖTH, Yves, 12683 Berlin (DE)
(74) Vertreter: Jungblut & Seuss
(86) Internationale Anmeldenummer: PCT/DE2014/000019
(87) Internationale Veröffentlichungsnummer: WO 2014/111085

(56) Entgegenhaltungen:
- EP-A1- 2 075 026
- EP-A2- 1 386 629
- WO-A1-2012/135912
- AU-A1- 2008 202 098
- US-A1- 2004 102 731
- US-A1- 2006 113 690

## Beschreibung

Die vorliegende Erfindung betrifft eine Vorrichtung zur Gaserwärmung und - befeuchtung für die Laparoskopie unter Verwendung von magnetischer Induktion. Die vorliegende Patentanmeldung nimmt die Priorität der deutschen Voranmeldung DE 102013000491.8 (Anmeldetag: 15.1.2013) in Anspruch.

### Stand der Technik

Die Laparoskopie ist ein medizinischer Eingriff bei dem die Bauchhöhle und die darin liegenden Organe visuell überprüft werden können. Hierzu werden üblicherweise kleine Hautschnitte (0,3 - 2 cm) in die Bauchdecke gemacht und durch diese ein Trokar eingebracht, welcher wiederum eine optische Vorrichtung aufnehmen kann. Mit Hilfe eines speziellen Endoskops (Laparoskop) kann der Bauchraum eingesehen werden. Bei der diagnostischen Laparoskopie wird der Bauchraum lediglich visuell inspiziert, im Rahmen eines therapeutischen Vorgriffs können auch operative Eingriffe vorgenommen werden.

Üblicherweise wird zu Beginn der Laparoskopie zunächst der Bauchraum mit Gas befüllt, um ein Pneumoperitoneum zu schaffen. Hierzu sind bereits verschiedene Gase verwendet worden, wie zum Beispiel Luft, Stickstoff oder Kohlendioxid (CO₂). Die Verwendung von Kohlendioxidgas hat sich besonders gut bewährt. Es wurde festgestellt, dass es, insbesondere bei längeren laparoskopischen Eingriffen sinnvoll ist, das eingeführte Gas einerseits zu erwärmen und andererseits zu befeuchten. Die Gaserwärmung dient dazu, den Patienten nicht abzukühlen, sowie ein diffuses Schmerzgefühl des Patienten zu vermeiden, welches wahrscheinlich eine Folge lokaler Abkühlung in Folge des Eintritts von kaltem Gas ist. Die Befeuchtung dient dazu, einem Austrocknen der inneren Bauchoberflächen vorzubeugen, auch um die dabei entstehende Abkühlung zu vermeiden. Für den erfindungsgemäßen Zweck sollten relative Gasfeuchtigkeiten von über 90% erreicht werden. , Dabei ergibt sich bei der Anwendung in der Laparoskopie die Besonderheit, dass die Volumenströme stark schwanken. So kann ein durchschnittlicher Gasfluss von 1-3 l/min. angenommen werden. Sollte jedoch eine größere Leckage auftreten, beispielswiese durch Aktivieren einer Absaugung, werden sofort Gasflussraten von > 20 l/min. gefordert, wobei auch diese den geforderten Feuchtigkeitswert von mehr als 90% erreichen sollen.

Hierzu sind im Stand der Technik bereits Anregungen gegeben. In der US 6,068,609 ist beispielsweise eine Vorrichtung beschrieben, die eine Gaserwärmung und -befeuchtung für die Laparoskopie ermöglicht. Hierin wird eine separate Kammer beschrieben, die mit einer Widerstandheizung ausgestattet ist. In der Kammer befindet sich zusätzlich ein absorbierendes Material, wie zum Beispiel ein Schwamm, der befeuchtet werden kann. Durch elektrische Beheizung der Kammer erfolgt dann ein Verdampfen des Wassers aus dem Befeuchtungsmittel. Die beschriebene Vorrichtung hat den Nachteil, dass ein elektrischer Anschluss erforderlich ist, um die Widerstandsheizung betreiben zu können. Dies erfordert einerseits einen gewissen Aufwand in der Herstellung, beispielsweise bei der Kabelführung und andererseits einen entsprechenden finanziellen Aufwand bei der Herstellung der Kammer. Da die Kammer einen Einwegartikel darstellt, sind geringerer Aufwand, insbesondere geringerer finanzieller Aufwand von besonderer Bedeutung für die Praxis. Weiterhin soll die Erfindung es möglichen, die Kammer nicht patientenseitig, sondern geräteseitig zu positionieren. Damit soll die Handhabung des Insufflationsschlauches verbessert werden. Aufgrund der Größe und des Gewichts der Kammer kann diese im nahen Operationsfeld den Arzt stören. Das Dokument DE 10 2005007773 A1 beschreibt eine Vorrichtung zur Befeuchtung von Atemgas, wobei in einer speziellen Ausführungsform ein Wasservorratsbehälter induktiv beheizt wird. Weiterer Stand der Technik wird gebildet durch die Druckschriften EP 1507568 B1, EP 1558877 B1 sowie EP 0827417 B1 und US 2004/0102731 A1. Aufgabe der vorliegenden Erfindung ist es daher, eine einfachere Vorrichtung zur Gaserwärmung und -befeuchtung zur Verfügung zu stellen, die die oben genannten Nachteile vermeidet.

### Lösung der Aufgabe

Die Lösung dieser Aufgabe erfolgt durch den Gegenstand des Patentanspruchs 1. Vorteilhafte Ausgestaltungen sind Gegenstand der Unteransprüche.

Kern der vorliegenden Erfindung ist die Gestaltung eines Heizelementes für die Gaserwärmung und -befeuchtung, die eine magnetische Induktion erlaubt. Induktive Erwärmung (oder auch Induktionsheizung mittels Wirbelströmen) ist ein bekanntes Verfahren, das an dieser Stelle nicht weiter erläutert werden muss. Hierzu wird eine von nieder- oder mittelfrequentem Wechselstrom (Beispiel: Frequenz: ∼50-200 kHz, Spannung: 10-50V, Strom: 1- 5 A) durchflossene Spule mit einer Oberfläche von z.B. 750 - 3.000 mm² in die Nähe eines elektrisch leitenden Materials gebracht, wodurch in diesem Körper ein Wirbelstrom induziert wird, welcher die Beheizung des genannten Körpers bewirkt. Die dabei entstehende Wärme entsteht unmittelbar in dem Körper, wird also nicht durch Wärmeleitung übertragen. Die induktive Erwärmung kann durch nicht leitende Materialien hindurch erfolgen, wie beispielsweise durch ein Kunststoffmaterial, in dem der Heizkörper angebracht ist. Der Wirkungsgrad hängt vom gewählten Material ab, je besser das Material leitet, desto schlechter ist im Allgemeinen der Erwärmungseffekt. Im Rahmen der vorliegenden Erfindung werden insbesondere Heizkörper aus Eisen verwendet, beispielsweise ein Quader in der Größe 30 x 45 x 2,5 mm (d.h. einer der Spule zugewandte Oberfläche von 1.350 mm²) mit einem Abstand zur Spule von ca. 3 mm. Dabei wird der Heizkörper komplett durchgewärmt, so dass zur Erwärmung des Gases sämtliche Körperflächen zur Verfügung stehen, im vorliegenden Beispiel daher ca. 3.075 mm². Sinnvollerweise wird das Heizelement eine Dicke von 2-5 mm aufweisen. Ein besonderer Vorteil der vorliegenden Erfindung ist, dass das Heizelement fast beliebige geometrische Formen annehmen kann und so einfach an den zur Verfügung stehenden Raum angepasst werden kann. Es versteht sich dabei von selbst, dass die Spule eine entsprechende Form und entsprechende Abmessungen haben muss. Ein weiterer Vorzug der Erfindung ist, dass keinerlei elektrische Leitungen in den Schlauch geführt werden müssen. Ferner können mehrere Heizelemente verwendet werden.

Erfindungsgemäß ist das Heizelement vorzugsweise gepaart mit einem Befeuchtungsmittel. Es handelt sich dabei um ein poröses Material welches eine möglichst hohe Wasseraufnahmekapazität aufweist und durch Zugabe von Wasser befeuchtet werden kann. Das Befeuchtungsmaterial sollte in der Nähe des Heizelementes angebracht werden. Es kann beispielsweise vollflächigen Kontakt mit dem Heizelement haben. In einer alternativen Ausführungsform besteht ein Abstand zwischen den einander gegenüberliegenden Oberfläche des Heizelement und der Oberfläche des Befeuchtungsmaterials. Bei den üblichen Dimensionen laparoskopischer Instrumente, insbesondere der Schläuche und deren Anschlusselemente, wird der Abstand 5 cm üblicherweise nicht übersteigen. Vorzugsweise wird der Abstand des Befeuchtungsmaterials 0-1 cm betragen, besonders bevorzugt 1-5 mm Als poröses Material kann im einfachsten Fall ein Gewebe aus Baumwolle (z.B. eine Mullbinde) verwendet werden, die in der Lage ist eine gewisse Menge Wasser aufzunehmen. Alternativ und/oder ergänzend können folgende Materialien verwendet werden: Schwämme, superabsorbierende Polymere (SAP), Löschpapier, Material aus Phenolharzen.

Wichtig ist, dass der Gasstrom möglichst so geführt wird, dass ein intensiver Kontakt des Gases mit dem Heizelement und ggf. dem Befeuchtungsmaterial ermöglicht wird. Beispielsweise kann das Gas durch kleinere Löcher in einer Eisenplatte geführt werden. Auf der anderen Seite der Eisenplatte kann dann das Befeuchtungsmaterial angebracht werden, welches direkt durchströmt wird. Alternativ kann das Gas auch an der Heizplatte vorbei geführt werden und danach mit dem Befeuchtungsmaterial in Kontakt treten. Die Abstände von Befeuchtungsmaterial und Eisenplatte haben naturgemäß Einfluss auf die Effektivität der Befeuchtung. Insofern sollten sie, wie oben beschreiben so nah wie möglich aneinander positioniert sein, z.B. 0-5 cm, vorzugsweise 0-1 cm, besonders bevorzugt 1-5 mm. Dabei kann das Heizelement möglichst vollflächig Kontakt mit dem Befeuchtungsmaterial haben, in vielen Fällen ist aber ein geringer Abstand wünschenswert.

Die Wasseraufnahmekapazität des porösen Materials ist naturgemäß abhängig von dem jeweiligen Material. Für eine normale Operation werden ca. 200 Liter Gas verbraucht. Um dieses auf annähernd 100% relative Feuchte zu befeuchten werden ∼10 ml Flüssigkeit benötigt. Es ist vorteilhaft, wenn die Menge des verwendeten Befeuchtungsmaterials diese Flüssigkeitsmenge aufnehmen kann.

Je nach geplanter Dauer des laparoskopischen Eingriffes und dem Gasfluss kann es ausreichend sein, das poröse Material einmal vor der Laparoskopie zu befeuchten. Das Befeuchtungsmittel wird vor Beginn des laparoskopischen Eingriffs mit Wasser befeuchtet. Insbesondere bei länger andauernden Operationen kann eine weitere Befeuchtung nötig sein. Hierzu kann die Vorrichtung eine optionale Zuleitung vorsehen, welche die weitere Einbringung von Wasser ermöglicht. Für den Fachmann auf dem Gebiet versteht sich von selbst, dass sowohl das vor Beginn der Operation, als auch ggf. während der Operation gegebenes Wasser steril sein muss.

Durch die unmittelbare räumliche Nähe zwischen dem induktiv erwärmten Heizelement und dem Befeuchtungsmittel wird im Rahmen des Gasflusses eine konstante Befeuchtung des Gases erzielt.

Selbstverständlich kann die erfindungsgemäße Vorrichtung nach dem Heizelement einen Temperatursensor enthalten, der die dem Patienten zugeführte Gastemperatur überwacht.

Da das Gas auf dem Weg durch den Insufflationsschlauch nach dem Befeuchten sich wieder auf die Umgebungstemperatur abkühlt, kann dieser ebenfalls beheizt werden. Damit kann das bereits erwärmte und befeuchtete Gas zum Patienten transportiert werden. Diese Schlauchheizung kann beispielsweise eine Widerstandheizung sein, welche auch über eine Temperatursonde verfügen kann.

Durch die Erfindung kann die Vorrichtung zum Befeuchten am geräteseitigen Ende des Insufflationsschlauches positioniert werden. Damit stört die Vorrichtung nicht im patientennahen Umfeld den operierenden Arzt.

Durch den Einsatz der Induktionstechnologie können die Herstellkosten des Einwegschlauches deutlich gesenkt werden. Zum einen entfallen Bauteile wie beispielsweise elektrische Kontakte, zum anderen verkürzt sich damit die Produktionszeit und somit die Kosten.

Die vorliegende Erfindung wird durch das nachfolgende Beispiel weiter illustriert, ohne dass dieses einschränkend wirken soll. Dem Fachmann wird nach dem Studium der vorliegenden Unterlagen weitere vorteilhafte Ausgestaltungen der Erfindung realisieren können, ohne erfinderisch tätig werden zu müssen.

### Beispiel:

Ein Heizelement bestehend aus Eisen, geformt als Platte in der Größe von 45x30x3mm wird in eine Kammer mit den Innenmaßen 30x45x18mm, bestehend aus biokompatiblem Kunststoff positioniert. Das Heizelement wird mit mehreren Löchern versehen, durch die das Gas beim Insufflieren durchströmt. Der verbleibende Raum der Kammer wird mit Mullbinde (Material: Baumwolle) ausgefüllt. Abschließend wird ein hydrophober Filter mit den Maßen 45x30mm positioniert.

Die auf diese Weise gebildete Kammer wird an einer Seite an einen Gasvorrat angeschlossen, an der anderen Seite mit einem Schlauch versehen, der in einer Veress-Nadel endet.

Ein Zugang zur Kammer ermöglicht das Befeuchten des Befeuchtungsmaterials mit sterilem Wasser.

Am Ausgang der Kammer wird in dem danach angebrachtem Schlauch eine Temperatursonde angeordnet. Damit kann die Ausgangstemperatur des Gases aus der Kammer gesteuert werden. Im weiteren Verlauf des Schlauches wird ein Heizdraht positioniert, der die Gastemperatur innerhalb des Schlauches konstant hält.

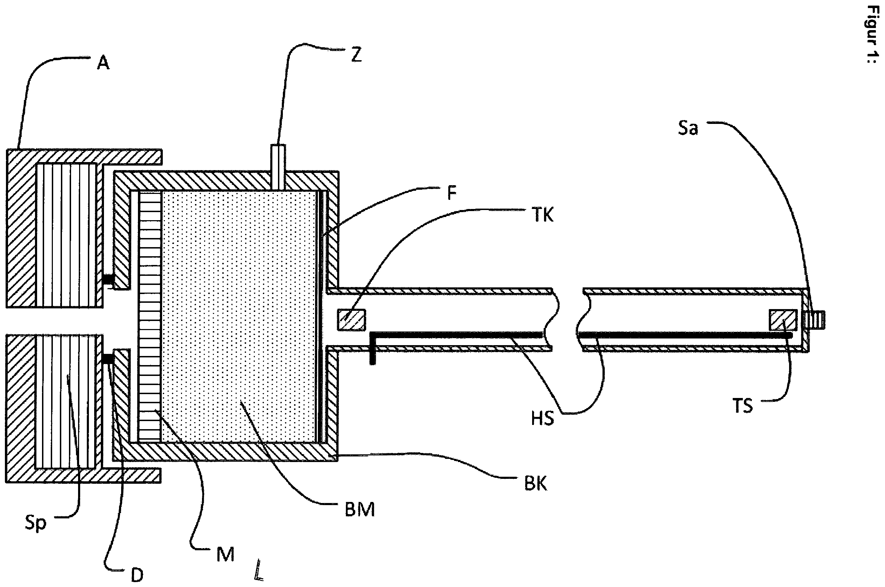

Die für die Induktion benötige Spule inklusive der dafür notwendigen Elektronik ist in dem Insufflator verbaut und nicht Teil des Schlauchsets. Die beschriebene Kammer wird in eine Vorrichtung am Gerät eingespannt, welche den Induktionsbereich enthält (dies wird in Figur 1 näher illustriert).

Das Befeuchtungsmittel wird vor Beginn des Eingriffs mit 10 ml Wasser benetzt. Durch die beschriebene Vorrichtung können bis zu 50 l CO₂ pro Minute geleitet werden. Die Heizleistung wird dabei über das magnetische Wechselfeld an den jeweiligen Bedarf angepasst, d. h. bei Absinken der Temperatur am Gasausgang der Kammer wird eine höhere Heizleistung eingestellt, bei Erhöhung der Temperatur wird die Heizleistung verringert. Die Gastemperatur in dem nach der Kammer angeordnetem Insufflationsschlauch wird auf ca. 37 °C konstant gehalten. Zur Induktion des Wechselfeldes wird eine Spannung von 24 Volt mit einer Frequenz von ca. 120 kHz angelegt. Dabei wird eine Heizleistung von bis zu 60 Watt erreicht. Die relative Gasfeuchtigkeit erreicht dabei Werte von ca. 95%.

### Bezugszeichenliste zu Figur 1:

- A: Aufnahme am Gerät für Insufflationsschlauch
- Z: Zuleitung für Befeuchtung des porösen Materials
- F: Filter
- TK: Temperaturmessung nach Kammer
- Sa: Schlauchanschluss patientenseitig
- TS: Temperaturmessung am Schlauchende
- HS: Heizdraht im Schlauch
- BK: Befeuchtungskammer
- BM: Befeuchtungsmittel
- M: Metallplatte mit Löchern
- D: Dichtung
- Sp: Spule

## Patentansprüche

1. Vorrichtung zur Heizung eines Gasstroms zur Verwendung in der Laparoskopie,
wobei der Gasstrom durch ein Heizelement (M) geheizt wird, ,
wobei das Heizelement (M) induktiv beheizt wird und
wobei das Heizelement Löcher aufweist, durch die der Gasstrom führt,
wobei der Gasstrom zusätzlich zum Heizelement (M) über ein Befeuchtungsmittel (BM) geführt wird,
wobei das Befeuchtungsmaterial einen Abstand zum Heizelement von 0-5 cm aufweist,
wobei das Heizelement (M) und das Befeuchtungsmittel (BM) Bestandteile einer separaten Kammer (BK) sind, durch die der Gasstrom geleitet wird,
wobei die Vorrichtung am geräteseitigen Ende (A) des Insufflationsschlauches positioniert ist.

2. Vorrichtung gemäß Anspruch 1, **dadurch gekennzeichnet, dass** die Kammer (BK) einen Zugang (Z) zum Befeuchten des Befeuchtungsmittels BM enthält.

3. Vorrichtung gemäß Anspruch 1 oder 2, **dadurch gekennzeichnet, dass** die Kammer (BK) am patientenseitigen Ausgang (Sa) einen hydrophoben Filter F enthält.

4. Vorrichtung gemäß mindestens einem der Ansprüche 1-3, **dadurch gekennzeichnet, dass** eine Temperatursonde (TK) die Gasaustrittstemperatur überwacht.

5. Vorrichtung gemäß mindestens einem der Ansprüche 1-4, **gekennzeichnet durch**, einen beheizten Schlauch am Kammerausgang.

6. Vorrichtung gemäß Anspruch 5, **dadurch gekennzeichnet, dass** der Schlauch durch einen Heizdraht (HS) beheizt wird.

7. Vorrichtung gemäß Anspruch 6, **dadurch gekennzeichnet, dass** eine Temperatursonde (TS) die Gasaustrittstemperatur überwacht.

8. Vorrichtung gemäß mindestens einem der Ansprüche 1 bis 7 , wobei das Heizelement (M) aus Eisen besteht.

9. Vorrichtung gemäß mindestens einem der Ansprüche 1 bis 8 , wobei die der Spule (Sp) zugewandte Oberfläche des Heizelementes (M) von 750 bis 3.000 mm² beträgt.

10. Vorrichtung gemäß mindestens einem der Ansprüche 1 bis 9, wobei das Befeuchtungsmittel (BM) Baumwollgewebe, Schwämme, superabsorbierende Polymere (SAP), Löschpapier oder Material aus Phenolharzen enthält.

## Claims

1. A device for heating a gas flow for use in laparoscopy,
wherein the gas flow is heated by a heating element (M),
wherein the heating element (M) is inductively heated, and wherein the heating element includes holes, through which the gas flow is conducted,
wherein the gas flow in addition to the heating element (M) is conducted over a humidifying material (BM),
wherein the humidifying material has a distance to the heating element of 0 to 5 cm,
wherein the heating element (M) and the humidifying material (BM) are components of a separate chamber (BK), through which the gas flow is conducted,
wherein the device is positioned at the device-side end (A) of the insufflation hose.

2. The device of claim 1, **characterized by** that the chamber (BK) contains an access (Z) for humidifying the humidifying material (BM).

3. The device of claim 1 or 2, **characterized by** that the chamber (BK) comprises a hydrophobic filter (F) at the patient-side outlet (Sa).

4. The device of at least one of claims 1 to 3, **characterized by** that a temperature probe (TK) monitors the temperature of the gas outlet.

5. The device of at least one of claims 1 to 4, **characterized by** a heated hose at the chamber outlet.

6. The device of claim 5, **characterized by** that the hose is heated by a heating wire (HS).

7. The device of claim 6, **characterized by** that a temperature probe (TS) monitors the temperature of the gas outlet.

8. The device of at least one of claims 1 to 7, wherein the heating element (M) is made of iron.

9. The device of at least one of claims 1 to 8, wherein the surface area of the heating element (M) facing the coil (Sp) is between 750 and 3,000 mm².

10. The device of at least one of claims 1 to 9, wherein the humidifying material (BM) contains cotton fabric, sponges, super-absorbing polymers (SAP), blotting paper, or material made of phenol resins.

## Revendications

1. Dispositif de chauffage d'un écoulement de gaz pour l'utilisation dans la laparoscopie,
dans lequel l'écoulement de gaz est chauffé par un élément de chauffage (M),
dans lequel l'élément de chauffage (M) est chauffé par induction, et dans lequel l'élément de chauffage comporte des trous, à travers lesquels l'écoulement de gaz est conduit,
dans lequel l'écoulement de gaz en addition à l'élément de chauffage (M) est conduit sur un moyen d'humidification (BM),
dans lequel le moyen d'humidification a une distance de l'élément de chauffage de 0 à 5 cm,
dans lequel l'élément de chauffage (M) et le moyen d'humidification (BM) sont des composants d'une chambre séparée (BK), à travers laquelle l'écoulement de gaz est conduit,
dans lequel le dispositif est positionné à l'extrémité (A) du tuyau d'insufflation se trouvant au côté du dispositif.

2. Dispositif selon la revendication 1, **caractérisé en ce que** la chambre (BK) comporte un accès (Z) pour l'humidification du moyen d'humidification (BM).

3. Dispositif selon la revendication 1 ou 2, **caractérisé en ce que** la chambre (BK) comprend un filtre hydrophobique (F) à la sortie (Sa) se trouvant au côté du patient.

4. Dispositif selon au moins une des revendications 1 à 3, **caractérisé en ce qu'**une sonde de température (TK) surveille la température de la sortie de gaz.

5. Dispositif selon au moins une des revendications 1 à 4, caractérisé en un tuyau chauffé à la sortie de la chambre.

6. Dispositif selon la revendication 5, **caractérisé en ce que** le tuyau est chauffé par un fil de chauffage (HS).

7. Dispositif selon la revendication 6, **caractérisé en ce qu'**une sonde de température (TS) surveille la température de la sortie de gaz.

8. Dispositif selon au moins une des revendications 1 à 7, dans lequel l'élément de chauffage (M) est en fer.

9. Dispositif selon au moins une des revendications 1 à 8, dans lequel la surface de l'élément de chauffage (M) en vis-à-vis de la bobine (Sp) est entre 750 et 3.000 mm².

10. Dispositif selon au moins une des revendications 1 à 9, dans lequel le moyen d'humidification (BM) comporte du tissu de coton, des éponges, des polymères superabsorbants (SAP), du papier buvard, ou un matériau en des résines phénoliques.
